# EUROPÄISCHE PATENTSCHRIFT

(11) **EP 1 339 734 B1**
(45) Veröffentlichungstag und Bekanntmachung des Hinweises auf die Patenterteilung: **23.08.2006**
(21) Anmeldenummer: 01993608.7
(22) Anmeldetag: 09.11.2001
(51) Int. Cl.: C07J 41/00, C07J 1/00, C07J 21/00, C07J 71/00

(54) **VERFAHREN ZUR HERSTELLUNG VON 4-(17ALPHA-SUBSTITUIERTEN-3-OXOESTRA-4,9-DIEN-11BETA-YL) BENZALDEHYD-(1E ODER 1Z)-OXIMEN**
METHOD FOR THE PRODUCTION OF 4-(17ALPHA SUBSTITUTED 3-OXOESTRA-4,9-DIEN-11BETA-YL)BENZALDEHYD-(1E OR 1Z)-OXIMES
PROCEDE DE PRODUCTION DE 4-(3-OXOESTRA-4,9-DIEN-11BETA-YL)BENZALDEHYD-(1E OU 1Z)-OXIMES SUBSTITUEES EN (17ALPHA DE FORMULE GENERALE (I))

(30) Priorität: 10.11.2000 DE 10056676
(43) Veröffentlichungstag der Anmeldung: 03.09.2003
(73) Patentinhaber: Schering AG, 13353 Berlin (DE)
(72) Erfinder: SCHUBERT, Gerd, 07743 Jena (DE); RING, Sven, 07749 Jena (DE); ERHART, Bernd, 07768 Kahla (DE)
(74) Vertreter: Ziebig, Marlene
(86) Internationale Anmeldenummer: PCT/DE2001/004218
(87) Internationale Veröffentlichungsnummer: WO 2002/038582

(56) Entgegenhaltungen:
- EP-A- 0 648 778
- C. RÜCKER ET AL: "Funktionalisierte Dioxide (syn-1,3) und Trioxide (syn,syn; syn,anti) des Tropilidens" CHEMISCHE BERICHTE., Bd. 117, Nr. 5, 1984, Seiten 1801-1833, XP002197491 VERLAG CHEMIE GMBH. WEINHEIM., DE ISSN: 0009-2940
- W. SEPPELT ET AL: "Funktionalisierte Dioxide (syn-1,4) und Trioxide (anti,anti) des Tropilidens" CHEMISCHE BERICHTE., Bd. 117, Nr. 5, 1984, Seiten 1834-1855, XP002197492 VERLAG CHEMIE GMBH. WEINHEIM., DE ISSN: 0009-2940
- KUWAHARA S ET AL: "SYNTHESIS OF LEVO PERIPLANONE-B A SEX PHEROMONE COMPONENT OF THE AMERICAN COCKROACH PERIPLANETA-AMERICANA" TETRAHEDRON, Bd. 46, Nr. 24, 1990, Seiten 8075-8082, XP002197493 ISSN: 0040-4020
- T. K. DHAR ET AL: "Structure of phaseolinone, a novel phytotoxin from Macrophomia phaseolina" TETRAHEDRON LETTERS., Bd. 23, Nr. 51, 1982, Seiten 5459-5462, XP002197494 ELSEVIER SCIENCE PUBLISHERS, AMSTERDAM., NL ISSN: 0040-4039

## Beschreibung

Es wird ein Verfahren zur Herstellung von 4-(17α-substituierten-3-oxoestra-4,9-dien-11β-yl)benzaldehyd-(1E oder 1Z)-oximen der allgemeinen Formel (I) 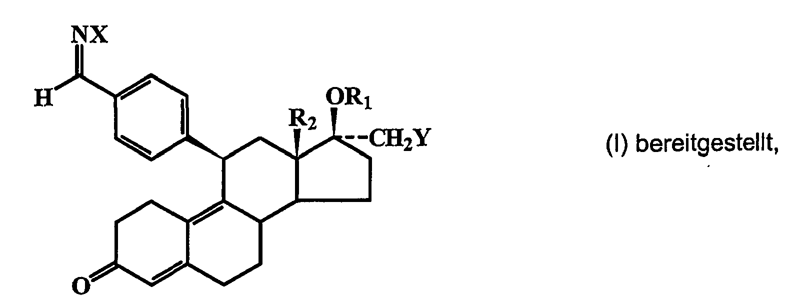 worin R₁ ein Wasserstoffatom, einen C₁₋₆-Alkylrest oder einen CₙF₂ₙ₊₁-Rest bedeutet, R₂ einen C₁₋₄-Alkylrest bedeutet, X eine E- oder Z-ständige OH-Gruppe bedeutet und Y eine OC₁₋₆-Alkylgruppe, SC₁₋₆-Alkylgruppe oder OCH₂CₙF₂ₙ₊₁-Gruppe bedeutet, wobei n 1, 2 oder 3 ist.
4-(17α-substituierte-3-oxoestra-4,9-dien-11β-yl)benzaldehyd-(1E oder 1 Z)-oxime sind bereits bekannt. Substanzen dieser Art sind in der Patentschrift DE 4332283 A1 (EP 0 648 778 B1) beschrieben. Die Verbindungen sind wegen der günstigen antigestagenen und geringen antiglucocorticoiden Wirkung von allgemeinem Interesse für die Behandlung einer Reihe von hormonabhängigen Erkrankungen der Frau wie beispielsweise der Endometriose.
Das bisherige Verfahren zu ihrer Herstellung geht von einem am C-3 als Ketal,
vorzugsweise dem als Dimethylketal geschützten 5α,10α-Epoxy-estr-9(11)-en-17-on (IIa) 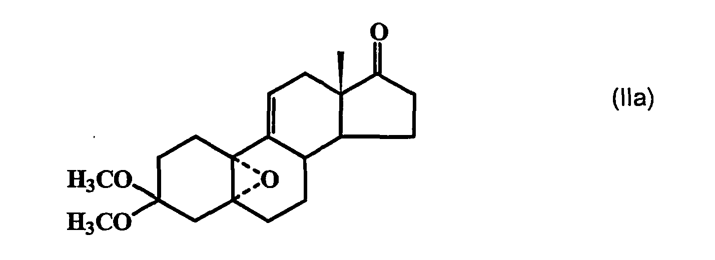 aus und erlaubt somit sowohl selektiv eine Vielzahl von verschieden 11β-Aryl-substituierten Steroiden als auch von unterschiedlichen 17α-substituierten Verbindungen herzustellen. In einem ersten Schritt wird das 5α,10α-Epoxid der Formel (IIa) durch eine durch Cu(I)Salze katalysierte Grignard-Reaktion mit einem 4-Brombenzaldehydketal, vorzugsweise dem 4-Brombenzaldehyddimethylketal, zu 11β-Arylsubstituierten 5α-Hydroxysteroiden der Formel (IIIa) 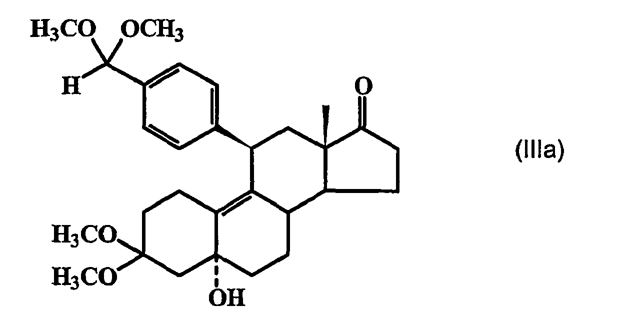 geöffnet. Die Ausbeuten des Verfahrens sind dabei nicht optimal, da auch ein Teil (3 bis 10 %) der 17-Oxogruppe angegriffen wird.

Es entstehen 11β,17α-Bisaryl-substituierte Steroide der Formel (IVa) 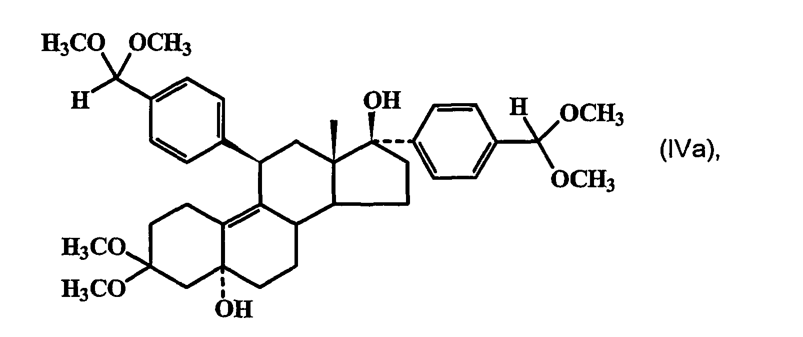 die sich nur aufwendig durch Chromatographie von den gewünschten 11β-Monoaryl-substituierten Verbindungen der Formel (IIIa) abtrennen lassen.
Das Gemisch der Verbindungen der Formel (IIIa) und (IVa) kann nach COREY und CHAYKOWSKY (J. Amer. chem. Soc. **84,** 3782 [1962]) hauptsächlich in das Spiroepoxid der Formel (Va) 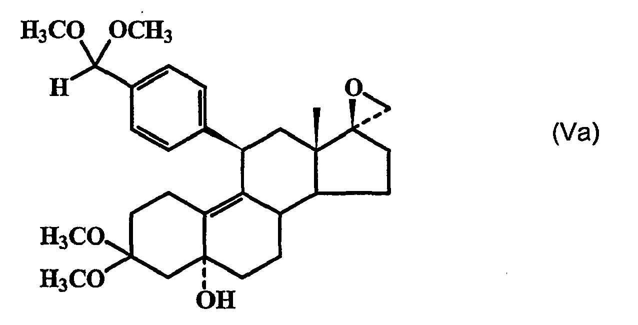 überführt werden, das durch Alkalimethylat zur 17α-Methoxyverbindung der Formel (VIa) 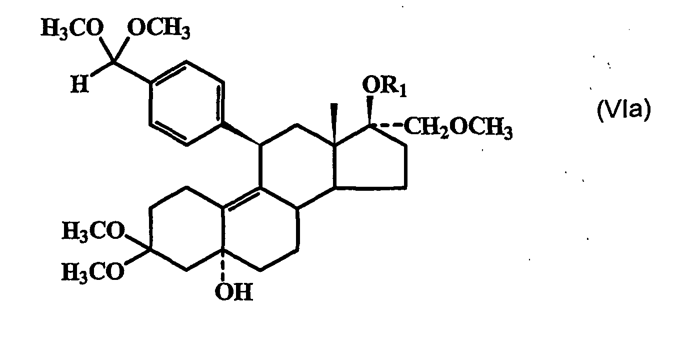 geöffnet wird, worin R₁ ein Wasserstoffatom ist und die entweder direkt oder nach Umsetzung der 17β-Hydroxyl mit Alkylhalogeniden in Gegenwart von Basen wie Kalium-tert.-butanolat in inerten Lösungsmitteln wie Tetrahydrofuran (THF) oder Toluol in die 17β-Ether der Formel (VIa), worin R₁ einen C₁₋₆-Alkylrest ist, durch saure Hydrolyse in die Benzaldehyde der Formel (Vlla) 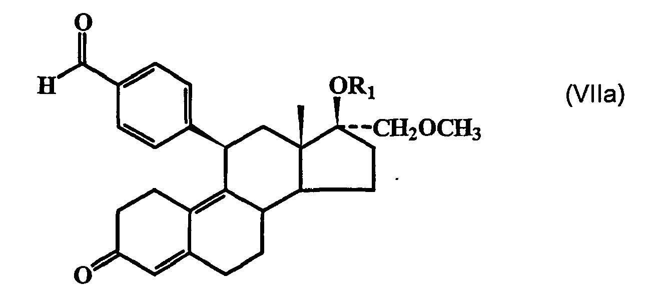 überführt wird, worin R₁ ein Wasserstoffatom oder ein C₁₋₆-Alkylrest ist. Die bei der Grignard-Reaktion als Nebenprodukte entstehenden 11β,17β-Bisarylsteroide der Formel (IVa) werden unter den genannten Bedingungen ständig mitgeführt und letztlich zu den 11β,17α-Bisaldehyden der Formel (VIIIa) 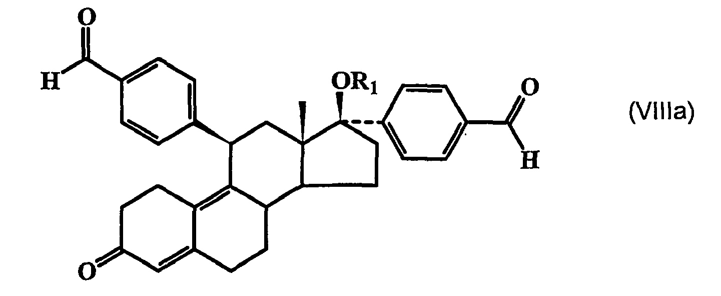 hydrolysiert, worin R₁ die vorstehend genannte Bedeutung hat. Diese Bisaldehyde der Formel (VIIIa) unterscheiden sich im Kristallisationsverhalten und in ihren chromatographischen Eigenschaften nur sehr geringfügig von den Monoaldehyden der Formel (VIIa) und sind nur schwer weitgehend quantitativ abzutrennen. Diese Nebenprodukte stören bei der Herstellung der erfindungsgemäßen Verbindungen der Formel (I).

Aufgabe der vorliegenden Erfindung ist deshalb ein effektiveres und technisch einfacheres Verfahren zur Herstellung von 4-(17α-substituierten-3-oxoestra-4,9-dien-11β-yl)benzaldehyd-(1E oder 1 Z)-oximen der allgemeinen Formel (I) zur Verfügung zu stellen, das den Angriff des Grignard-Verbindung am C-17 verhindert und dadurch mit höherer Ausbeute und Reinheit zu Verbindungen der Formel (I) führt.

Diese Aufgabe wird gemäß dem Verfahren nach Anspruch 1 gelöst.

Dadurch, daß die 17-Oxogruppe vor der Grignardierung in die gewünschte 17α-substituierte Verbindung überführt wird, wird die Bildung des Nebenprodukts der Formel (VIIIa) verhindert, wodurch die Zielverbindungen mit höherer Ausbeute und Reinheit erhalten werden. So kann beispielsweise ausgehend von der Verbindung (II) nach dem Verfahren gemäß DE 43 32 283 A1 der Aldehyd (VIIb) in ca. 5,6 % Ausbeute und entsprechend das Oxim (Ic) in ca. 3,8 % Ausbeute hergestellt werden. Durch das erfindungsgemäße Verfahren läßt sich der Aldehyd (Vllb) nunmehr in ca. 25 % Ausbeute bzw. das Oxim (Ic) in ca. 17 % Ausbeute aus dem Olefin (IX) herstellen, ohne daß spezielle chromatographische Bedingungen für die Reinigung angewendet werden müssen.
Bevorzugte Ausführungsformen der Erfindung sind in den Unteransprüchen angegeben. Wegen weiterer Vorteile der Erfindung wird auf die folgende Beschreibung und die Ausführungsbeispiele verwiesen.

Erfindungsgemäß werden die 3,3-Dimethoxy-5α,10α-epoxy-estr-9(11)-en-17-one der Formel (II) 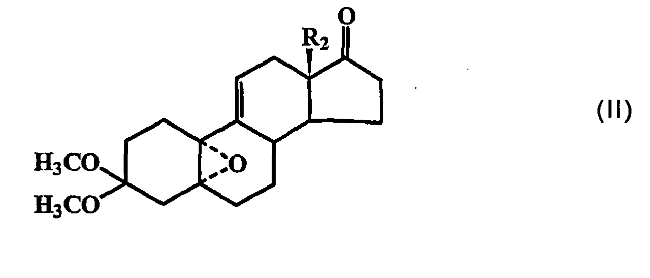 in der R₂ einen C₁₋₄-Alkylrest bedeutet, mit einem aktiven Methylenreagens, hergestellt beispielsweise aus Trimethylsulfoniumiodid und einer starken Base, wie Kalium-tert.-butanolat oder Kaliumhydroxid, in Lösungsmitteln, wie Dimethylsulfoxid (DMSO), Dimethylformamid (DMF) oder Toluol, in das 5α,10α-Epoxy-17(S)-Spiroepoxid der Formel (IX) 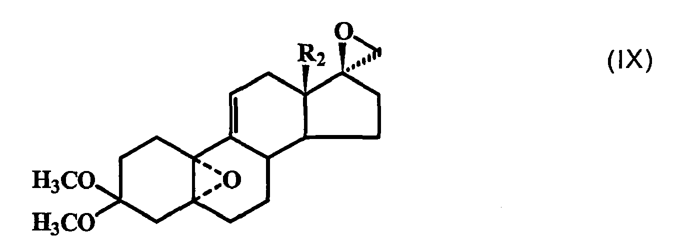 umgewandelt, wobei R₂ die vorstehend genannte Bedeutung besitzt, das nach regio- und stereoselktiver Spaltung der 17-Spiroepoxygruppe durch Alkali- oder Erdalkalialkoholat, vorzugsweise durch Natriummethanolat, durch Alkylmercaptane in Gegenwart von Alkalihydroxiden oder Kalium-tert.-butanolat, alternativ direkt mit Alkalimercaptiden oder mit Perfluoralkylalkoholen in Gegenwart von Alkali, vorzugsweise Kalium-tert.-butanolat, in Lösungsmitteln, wie Methanol, DMF oder DMSO, zu den 17α-substituierten Verbindungen der Formel (X) 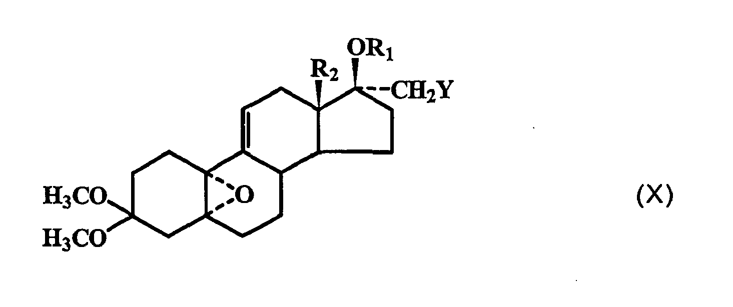 geöffnet wird, wobei Y eine OC₁₋₆-Alkylgruppe, SC₁₋₆-Alkylgruppe oder OCH₂CₙF₂ₙ₊₁-Gruppe bedeutet, wobei n 1, 2 oder 3 ist und R₁ ein Wasserstoffatom ist und R₂ die oben genannte Bedeutung besitzt, die wahlweise durch Umsetzung der 17β-Hydroxylgruppe mit Halogenalkylverbindungen oder Halogenalkylfluoriden (Halogen = Cl, Br oder I) in Gegenwart von starken Basen wie Kaliumhydroxid, Alkoholaten, wie Kalium-tert.-butanolat, Silberfluoriden, Alkalimetallen und Naphthalin oder Biphenyl in inerten Lösungsmitteln, wie Ethern, THF oder Toluol, in die 17β-Ether oder 17β-Fluoralkylether der Formel (X) umgewandelt werden, worin R₁ ein C₁₋₆-Alkylrest oder ein CₙF₂ₙ₊₁ - Rest ist, wobei n 1, 2 oder 3 ist, und R₂ und Y die oben genannte Bedeutung haben. Die Verbindungen der Formel (X) werden mit 4-Brombenzaldehydketalen, wie dem 4-Brombenzaldehyd-1,1-ethylenketal oder dem 4-Brombenzaldehyd-1,1-dimethylketal, Magnesium und Cu(I)Cl bei Temperaturen zwischen -35 °C und Raumtemperatur zu den entsprechenden 3,3-Dimethoxy-5α-hydroxy-11β-benzaldehydketalen der Formel (XI) 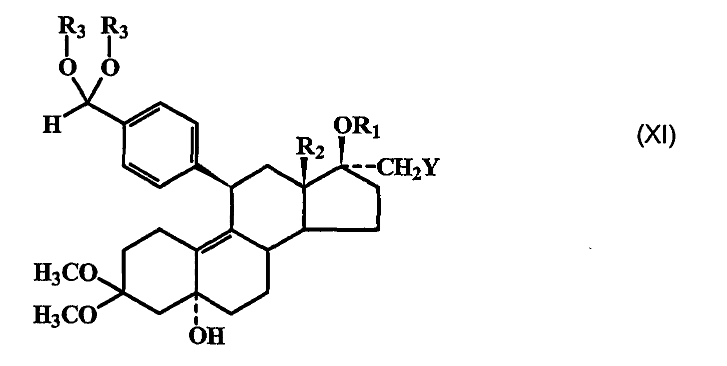 umgesetzt, worin R₃ für einen Methylrest oder eine Ethylidengruppe steht und R₁, R₂ und Y die vorstehend gegebene Bedeutung haben, die durch saure Hydrolyse der Schutzgruppen, beispielsweise mit verdünnnter Essigsäure oder p-Toluolsulfonsäure in Aceton, in die 11β-Benzaldehyd-Derivate der Formel (XII) überführt werden, 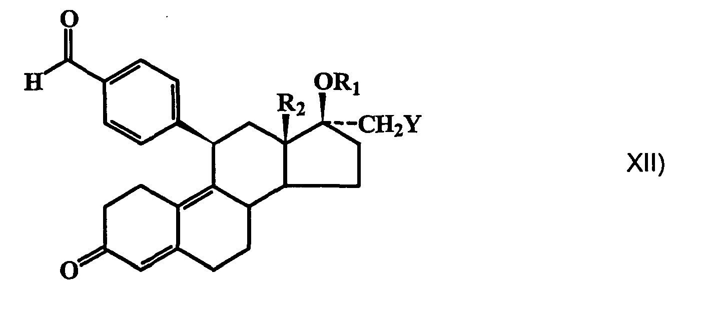 worin R₁, R₂ und Y die vorstehend gegebene Bedeutung haben und die Aldehydfunktion selektiv durch Hydroxylammoniumsalze, vorzugsweise Hydroxylaminhydrochlorid in Gegenwart von Basen, vorzugsweise Pyridin oder Triethylamin, bei Raumtemperatur in ein Gemisch der E/Z-Benzaldoxime der allgemeinen Formel (I) 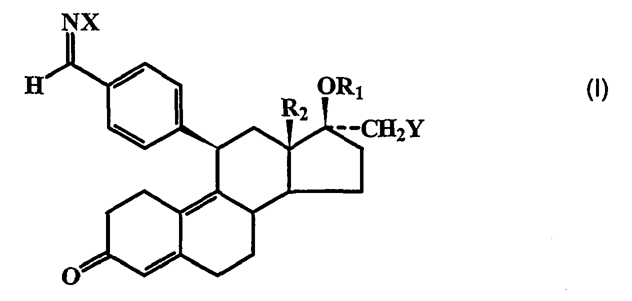 umgewandelt wird, worin X eine E- oder Z-ständige OH-Gruppe bedeutet, R₁, R₂ und Y die vorstehend gegebene Bedeutung haben, die umkristallisiert oder durch Chromatographie getrennt, gereinigt und als Einzelkomponenten isoliert werden.

Unter "Alkylrest" wird in der vorliegenden Erfindung ein verzweigter oder geradkettiger Alkylrest verstanden. Als C₁₋₄- bzw C₁₋₆-Alkylreste seien beispielsweise eine Methyl-, Ethyl-, n-Propyl-, i-Propyl-, n-Butyl, i-Butyl- oder tert.Butyl-, n-Pentyl-, i-Pentyl-, n-Hexyl-, 2-Methylpentyl-, 3-Methylpentyl-, 2,2-Dimethylbutyl- oder 2,3-Dimethylbutylgruppe genannt. Unter CₙF₂ₙ₊₁-Rest wird ein verzweigter oder geradkettiger Fluoralkylrest mit 1 bis 3 Kohlenstoffatomen verstanden, wobei Beispiele eine Trifluormethyl-, Pentafluorethyl-, Heptafluor-n-propyl- oder Heptafluor-iso-propylgruppe sind.

Bevorzugt bedeuten R₁ und R₂ einen C₁₋₃-Alkylrest, besonders bevorzugt eine Methylgruppe oder eine Trifluormethylgruppe. Y bedeutet bevorzugt einen OC₁₋₃-Alkyl- oder SC₁₋₃-Alkylrest, besonders bevorzugt eine Methoxy-, Ethoxy-, Isopropyloxy-, Methylthio- oder Ethylthiogruppe, oder Trifluorethoxygruppe. Die Verbindungen der Formel (I), in denen R₁ ein CₙF₂ₙ₊₁-Rest ist und/oder Y eine OCH₂CₙF₂ₙ₊₁-Gruppe ist, sind neu.
Am stärksten bevorzugt sind im Rahmen von Verbindungen der Formel (I) die folgenden Verbindungen:
4-[17β-Hydroxy-17α-(methoxymethyl)-3-oxoestra-4,9-dien-11β-yl]benzaldehyd-1 E-oxim,
4-[17β-Hydroxy-17α-(methoxymethyl)-3-oxoestra-4,9-dien-11β-yl]benzaldehyd-1 Z-oxim,
4-[17β-Methoxy-17α-(methoxymethyl)-3-oxoestra-4,9-dien-11β-yl]benzaldehyd-1 E-oxim,
4-[17β-Methoxy-17α-(methoxymethyl)-3-oxoestra-4,9-dien-11β-yl]benzaldehyd-1 Z-oxim,
4-[17β-Ethoxy-17α-(methoxymethyl)-3-oxoestra-4,9-dien-11β-yl]benzaldehyd-1 E-oxim,
4-[17β-Hydroxy-17α-(ethoxymethyl)-3-oxoestra-4,9-dien-11β-yl]benzaldehyd-1 E-oxim,
4-[17β-Methoxy-17α-(ethoxymethyl)-3-oxoestra-4,9-dien-11β-yl]benzaldehyd-1 E-oxim,
4-[17β-Hydroxy-17α-(isopropyloxymethyl)-3-oxoestra-4,9-dien-11β-yl]benzaldehyd-1 E-oxim,
4-[17β-Methoxy-17α-(isopropyloxymethyl)-3-oxoestra-4,9-dien-11β-yl]benzaldehyd-1E-oxim,
4-[17β-Hydroxy-17α-(ethylthiomethyl)-3-oxoestra-4,9-dien-11β-yl]benzaldehyd-1 E-oxim und
4-[17β-Hydroxy-17α-(1,1,1-trifluorethoxymethyl)-3-oxoestra-4,9-dien-11 β-yl]benzaldehyd-1E-oxim.

Die Verbindungen werden gut am Gestagenrezeptor gebunden, zeigen im Tierexperiment eine starke antigestagene Aktivität, besitzen eine partielle gestagene Aktivität und weisen nur eine geringe glucocorticoide Rezeptorbindung auf (DE 4332283 A1 (EP 0 648 778 B1)).

Die nachfolgenden Beispiele dienen zur näheren Beschreibung der Erfindung.

### Beispiel 1

### 4-[17β-Hydroxy-17α-(methoxymethyl)-3-oxoestra-4,9-dien-11β-yl]benzaldehyd-1E-oxim (Ia)

### Stufe a

25 g 3,3-Dimethoxy-5α,10α-epoxy-estr-9(11)-en-17-on (IIa) werden in 200 ml DMSO gelöst und mit 34 g Trimethylsulfoniumjodid versetzt. Anschließend gibt man 24 g festes Kalium-tert.-butanolat hinzu und rührt 3 Stunden bei Raumtemperatur, gießt in eiskalte wäßrige Ammoniumchlorid-Lösung ein, extrahiert mit Essigester, wäscht neutral, trocknet die organische Phase mit Natriumsulfat und engt im Vakuum ein. Man erhält 27 g 3,3-Dimethoxy-5α,10α-epoxy-estr-9(11)-en-17(S)-spiroepoxid (IXa) als klebrigen Schaum, der direkt in der nächsten Stufe eingesetzt wird.

### Stufe b

27 g 3,3-Dimethoxy-5α,10α-epoxy-estr-9(11)-en-17(S)-spiroepoxid (IXa) werden in 100 ml Methanol gelöst, unter Argon werden 100 ml 3N Natriummethylat-Lösung zugesetzt und die Mischung wird 2 Stunden unter Rückfluß erhitzt. Es wird abgekühlt, die Hälfte des Methanols wird abdestilliert und der Rest in Essigester aufgenommen, die Lösung wird mit Wasser versetzt und die Phasen werden getrennt. Die organische Phase wird neutral gewaschen, über Natriumsulfat getrocknet und im Vakuum eingeengt. Man erhält 29,5 g 3,3-Dimethoxy-17α-(methoxymethyl)-5α,10α-epoxy-estr-9(11)-en-17β-ol (Xa) als einen klebrigen Schaum, der direkt in der nächsten Stufe eingesetzt wird.

### Stufe c

10 g 3,3-Dimethoxy-17α-methoxymethyl-5α,10α-epoxy-estr-9(11)-en-17β-ol (Xa) in 50 ml abs. THF werden bei -35°C zu einer Grignard-Lösung, hergestellt aus 2,7 g Magnesium, 25 g 4-Brombenzaldehydethylenketal in 130 ml THF und 0,65 g Kupfer(I)chlorid, zugetropft. Man läßt nach 2 Stunden auf Raumtemperatur erwärmen, zersetzt mit wäßriger Ammoniumchlorid-Lösung und extrahiert die Lösung mit tert.-Butylmethylether. Die organische Phase wird neutral gewaschen, getrocknet und im Vakuum eingedampft. Man erhält 16 g eines Rohproduktes aus dem durch Chromatographie an Kieselgel das 4-(3,3-Dimethoxy-5α,17β-dihydroxy-17α-methoxymethyl-estr-9-en-11β-yl)benzaldehyd-1,1-ethylenketal (XIa) isoliert wird.
Schmp. 111 bis 116 °C. α_{D} = -5 ° (CHCl₃). ¹H-NMR: 7,36 (d, 2H, J=8,1, H3'), 7,24 (d, 2H, J=8,1, H2'), 5,76 (s,1H, PhCH), 4,67 (s, 1H, OH), 4,27 (d, 1H, J= 8,1, H-11α), 4,02-4,14 (m, 4H, Etylenketal), 3,38 (s, 3H, OCH₃), 3,22 (s, 3H, OCH₃), 3,21 (s, 3H, OCH₃), 3,17 (d, 1 H, J=9,0, CH₂O), 2,55 (s, 1H, OH), 0,46 (s, 3H, H-18),

### Stufe d

45 g 4-(3,3-Dimethoxy-17α-methoxymethyl-5α-hydroxy-estr-9-en-11β-yl)benzaldehyd-1,1-ethylenketal (Xla) werden in 100 ml tert.-Butylmethylether gelöst, mit 1,2 g p-Toluolsulfonsäure versetzt und 1 Stunde bei Raumtemperatur gerührt. Nach Zugabe von 15 ml Bicarbonat-Lösung wird mit Methylenchlorid extrahiert, die organische Phase neutral gewaschen, getrocknet und im Vakuum eingeengt. Nach Zugabe von tert.-Butylmethylether fällt der 4-[17β-Hydroxy-(17α-(methoxymethyl)-3-oxoestra-4,9-dien-11β-yl]benzaldehyd (VIIa) als Rohprodukt aus, das durch Chromatographie an Kieselgel gereinigt und aus Essigester umkristallisiert wird.
Schmp. 235 bis 240° C. α_{D} = +209° (CHCl₃). ¹H-NMR: 9,97 (s, 1H, - CHO), 7,80 (d, 2H, J = 8,1, H-3'), 7,38 (d, 2H, J = 8,1, H-2'), 5,80 (s, 1 H, H-4), 4,45 (d, 1 H, J = 7,5, H-11), 3,57 (d, 1H, J = 9,2, CH₂O), 3,42 (d, 1H, J = 10,8, CH₂O), 3,41 (s, 3H, OCH₃), 0,51 (s, 3H, H-18).

### Stufe e

33g 4-[17β-Hydroxy-17α-(methoxymethyl)-3-oxoestra-4,9-dien-11β-yl]benzaldehyd (Vlla) werden unter Argonschutz in 250 ml Pyridin gelöst und mit 5,8 g Hydroxylaminhydrochlorid versetzt. Nach 2 Stunden wird in Eiswasser eingerührt, der Niederschlag wird abgesaugt, gewaschen und getrocknet. Das Rohprodukt (40 g) wird durch Chromatographie an Kieselgel gereinigt. Man erhält 20 g 4-[17β-Hydroxy-(17α-(methoxymethyl)-3-oxoestra-4,9-dien-11β-yl]benzaldehyd-1E-oxim (la) [Schmp. 135 bis 145°C (EtOH/Wasser). α_{D} = +236° (CHCl₃). ¹H-NMR: 9,00 (s, 1H, NOH), 8,11 (s, 1H, HC=N), 7,45 (d, 2H, J = 8,2 , H-3'), 7,17 (d, 2H, J = 8,2 , H-2'), 5,79 (s, 1 H, H-4), 4,38 (d, 1H, J = 7,1, H-11), 3,58 (d, 1H, J = 9,0, CH₂O), 3,43 (s, 3H, OCH₂), 3,25 (d, 1H , J = 9,0, CH₂O), 0,48 (s, 3H, H-18)] und 1,5 g 4-[17β-Hydroxy-17α-(methoxymethyl)-3-oxoestra-4,9-dien-11β-yl]benzaldehyd-1Z-oxim (Ib) [Schmp. 135 bis 146°C (Aceton). α_{D} = +192°. ¹H-NMR: 8,56 (s, 1H, NOH), 7,86 (d, 2H, J = 8,4 , H-3'), 7,33 (s, 1 H, HC=N), 7,26 (d, 2H, J = 8,4 , H-2'), 5,79 (s, 1 H, H-4), 4,41 (d, 1 H, J = 7,2 , H-11), 3,57 (d, 1 H, J = 9,1 , CH₂O), 3,42 (s, 3H, OCH₃), 3,23 (d, 1H, J = 9,1, CH₂O), 0,54 (s, 3H, H-18).

### Beispiel 2

### 4-[17β-Methoxy-17α-(methoxymethyl)-3-oxoestra-4,9-dien-11β-yl]benzaldehyd-1E-oxim (Ic)

### Stufe a

29,5 g des in Beispiel 1, Stufe b hergestellten 3,3-Dimethoxy-17α-methoxymethyl-5α,10α-epoxy-estr-9(11)-en-17β-ols (Xa) werden unter Argon in 650 ml Toluol gelöst, mit 110 g Kalium-tert.-butanolat versetzt und bei Raumtemperatur gerührt. Man tropft 70 ml Methyljodid in 30 ml Toluol innerhalb von 2 Stunden zu. Danach wird mit Wasser verdünnt, die Phasen werden getrennt, die organische Phase neutral gewaschen getrocknet und im Vakuum eingedampft. Man erhält 30 g 3,3,17β-Trimethoxy-5α,10α-epoxy-estr-9(11)-en-17α-methoxy-methylether (Xb) als gelbes Harz, das mit Hexan behandelt wird.
Schmp. 114 bis 118°C (Hexan). α_{D} = +7° (CHCl₃). ¹H-NMR: 6,00 (m, 1H, H-11), 3,57 (d, 1 H, J = 10,3 , CH₂O), 3,36 (s, 3H, OCH₃), 3,30 (d, 1H, J = 10,3, CH₂O), 3,28 (s, 3H, OCH₃), 3,19 (s, 3H, OCH₃), 3,13 (s, 3H, OCH₃), 0,88 (s, 1 H, H-18).

### Stufe b

Zu einer Grignard-Lösung, hergestellt aus 24 g 4-Brombenzaldehydethylenketal und 2,0 g Magnesium in 140 ml THF, werden bei -35 °C 500 mg Kupfer(I)chlorid zugegeben. Man rührt 20 Minuten bei dieser Temperatur und tropft eine Lösung von 10 g 3,3,17β-Trimethoxy-5α,10α-epoxy-estr-9(11)-en-17α-methoxymethylether (Xb) in 40 ml THF zu. Anschließend läßt man die Reaktionslösung auf Raumtemperatur erwärmen, zersetzt den Ansatz mit wäßriger Ammoniumchlorid-Lösung, extrahiert die Lösung mit Essigester und wäscht die organische Phase mit Wasser, trocknet sie mit Natriumsulfat und engt die Lösung im Vakuum ein. Das Rohprodukt, 4-(3,3-Dimethoxy-5α-hydroxy-17β-methoxy-17α-methoxymethyl-estr-9-en-1 1β-yl)benzaldehyd-1,1-ethylenketal (XIb), (15 g) wird direkt in der nächsten Stufe eingesetzt.

### Stufe c

15 g Rohprodukt 4-(3,3-Dimethoxy-5α-hydroxy-17β-methoxy-17α-methoxymethyl-estr-9-en-11β-yl)benzaldehyd-1,1-ethylenketal (Xlb) werden in 120 ml Aceton gelöst und mit 1,3 g p-Toluolsulfonsäure versetzt. Nach 1 Stunde wird neutralisiert und mit Wasser verdünnt. Dabei fällt der 4-[17β-Methoxy-17α-methoxymethyl)-3-oxoestra-4,9-dien-11β-yl]benzaldehyd (VIIb) aus, der abgesaugt und in Aceton umkristallisiert wird.
Schmp. 245 bis 250 °C (Aceton). α_{D} = +193° (CHCl₃). ¹H-NMR: 9,97 (s, 1H, CHO), 7,79 (d, 2H, J = 8,1, H-3'), 7,37 (d, 2H, J = 8,1, H-2'), 5,79 (s, 1 H, H-4), 4,44 (d, 1 H, J = 7,5, H-11), 3,56 (d, 1 H, J = 10,8, CH₂O), 3,42 (d, 1H, J = 10,8, -CH₂O), 3,41 (s, 3H, OCH₃), 3,25 (s, 3H, OCH₃), 0,51 (s, 3H, H-18).

### Stufe d

Zu einer Lösung von 10 g 4-[17β-Methoxy-17α-(methoxymethyl)-3-oxoestra-4,9-dien-11β-yl]benzaldehyd (Vllb) in 100 ml Pyridin werden bei Raumtemperatur 1,75 g Hydroxylaminhydrochlorid zugegeben und die Mischung wird 2 Stunden gerührt. Man gießt in Eiswasser ein, saugt den Niederschlag ab, trocknet mit Calciumchlorid und chromatographiert das Rohprodukt an Kieselgel. Man isoliert 7g 4-[17β-Methoxy-17α-(methoxymethyl)-3-oxoestra-4,9-dien-11β-yl]benzaldehyd-1E-oxim (Ic) [Schmp. 196 bis 198 °C (EtOH/H₂O). α_{D} = +220° (CHCl₃). ¹H-NMR: 8,38 (s, 1H, NOH), 8,10 (s, 1H, HC=N), 7,47 (d, 2H, J = 8,1, H-3'), 7,20 (d, 2H, J = 8,1, H-2'), 5,79 (s, 1H, H-4), 4,38 (d, 1 H, J = 7,3, H-11), 3,58 (d, 1H, J = 10,8, CH₂O), 3,41 (s, 3H, OCH₃), 3,41 (d, 1H, J = 10,8, CH₂O), 3,25 (s, 3H, OCH₃), 0,54 (s, 3H, H-18)] und 300 mg 4-[17β-Methoxy-17α-(methoxymethyl)-3-oxoestra-4,9-dien-11β-yl]benzaldehyde-1Z-oxim (Id) [Schmp. 120 bis 138 °C (Aceton/n-Hexan). α_{D} = +217° (CHCl₃). ¹H-NMR: 9,38 (s, 1 H, NOH), 7,88 (d, 2H, J = 8,9, H-3'), 7,33 (s, 1H, HC=N), 7,26 (d, 2H, J = 8,9, H-2'), 5,79 (s, 1 H, H-4), 4,39 (d, 1 H, J = 7,3, H-11), 3,58 (d, 1 H, J = 10,5, CH₂O), 3,42 (d, 1 H, J = 10,5, CH₂O), 3,41 (s, 3H, OCH₃), 3,26 (s, 3H, OCH₃), 0,54 (s, 3H, H-18).

### Beispiel 3

### 4-[17β-Ethoxy-17α-(methoxymethyl)-3-oxoestra-4,9-dien-11β-yl]benzaldehyd-1E-oxim (Ie)

### Stufe a

4,16 g des in Beispiel 1, Stufe b hergestellten 3,3-Dimethoxy-17α-methoxymethyl-5α,10α-epoxy-estr-9(11)-en-17β-ols (Xa) werden portionsweise mit insgesamt 15,6 g Kalium-tert.-butanolat und 76 ml lodethan in 405 ml Toluol bei 35 °C innerhalb von 14 h umgesetzt. Zur Aufarbeitung wird auf Raumtemperatur gekühlt und Wasser zugesetzt, wobei sich Salze lösen. Die Phasen werden getrennt und die wäßrige Phase mit Toluol nachextrahiert. Die Lösung wird gewaschen, getrocknet und eingedampft. Man erhält 4,2 g 3,3,-Dimethoxy-5α,10α-epoxy-17β-ethoxy-estr-9(11)-en-17α-methoxymethylether (Xc) als Rohprodukt, das direkt in der nächsten Stufe eingesetzt wird.

### Stufe b

Zu einer Grignard-Lösung, hergestellt aus 5 g 4-Brombenzaldehydethylenketal und 0,45 g Magnesium in 60 ml THF, wird bei -35 °C 0,1 g Kupfer(I)chlorid zugegeben. Man rührt 30 Minuten bei dieser Temperatur und tropft eine Lösung von 2,5 g 3,3-Dimethoxy-5α,10α-epoxy-17β-ethoxy-estr-9(11)-en-17α-methoxymethylether (Xc) in 15 ml THF zu. Anschließend läßt man die Reaktionslösung auf Raumtemperatur erwärmen, zersetzt den Ansatz mit wäßriger Ammoniumchlorid-Lösung und isoliert nach üblicher Aufarbeitung das 4-[3,3-Dimethoxy-5α-hydroxy-17β-ethoxy-17α-(methoxymethyl)-estr-9-en-11β-yl]benzaldehyd-1,1-ethylenketal (XIc) (4,5 g), das direkt in der nächsten Stufe eingesetzt wird

### Stufe c

4,5 g 4-[3,3-Dimethoxy-5α-hydroxy-17β-ethoxy-17α-(methoxymethyl)-estr-9-en-11β-yl]benzaldehyd-1,1-ethylenketal (Xlc) werden in 60 ml Aceton gelöst. Man fügt 1,6 g p-Toluolsulfonsäure zu und gießt nach 1 Stunde in Eiswasser ein. Der dabei ausfallende 4-[17β-Ethoxy-17α-methoxymethyl)-3-oxoestra-4,9-dien-11β-yl]benzaldehyd (VIIc) wird abgesaugt, getrocknet und aus Aceton/Hexan und erneut aus tert.-Butylmethylether umkristallisiert.
Schmp. 164 bis 167 °C. α_{D} = +199° (CHCl₃). ¹H-NMR: 9,97 (s, 1H, CHO), 7,80 (d, 2H, J = 8,1, H-3'), 7,37 (d, 2H, J = 8,1, H-2'), 5,79 (s, 1H, H-4), 4,43 (d, 1H, J = 7,5, H-11), 3,58 (d, 1H, J = 10,8, CH₂O), 3,41 (m, 2H, CH₂O), 3,40 (s, 3H, OCH₃), 1,10 (t, 3H, Ethyl), 0,51 (s, 3H, H-18).

### Stufe d

1,7 g 4-[17β-Ethoxy(17α-methoxymethyl)-3-oxoestra-4,9-dien-11β-yl]benzaldehyd (VIIc) werden in 25 ml Pyridin mit 250 mg Hydroxylaminhydrochlorid 1 Stunde bei Raumtemperatur gerührt. Danach gießt man in 100 ml Eiswasser ein, saugt den Niederschlag ab, wäscht mit Wasser neutral und trocknet mit Calciumchlorid. Das Rohprodukt (1,7 g) wird durch Chromatographie an Kieselgel gereinigt. Man erhält 890 mg 4-[17β-Ethoxy-17α-(methoxymethyl)-3-oxoestra-4,9-dien-11β-yl]benzaldehyd-1E-oxim (Ie).
Schmp. 184 bis 187 °C (Aceton/Hexan). α_{D} = +214° (CHCl₃). ¹H-NMR: 9,10 (s, 1H, CH=N), 7,58 (s, 1H, OH), 7,49 (d, 2H, J = 8,4, H-3'), 7,21 (d, 2H, J = 8,4, H-2'), 5,78 (s, 1H, H-4), 4,38 (d, 1 H, J = 6,9, H-11), 3,62 (d, 1 H, J = 10,8, CH₂O), 3,40 (s, 3H, OCH₃), 3,36 (d, 1H, J = 10,8, CH₂O), 1,11 (t, 3H, CH₂CH₃), 0,54 (s, 3H, H-18).

### Beispiel 4

### 4-[17β-Hydroxy-17α-(ethoxymethyl)-3-oxoestra-4,9-dien-11β-yl]benzaldehyd-1E-oxim (If)

Die Herstellung erfolgt gemäß Beispiel 1, Stufen 1b 1 c, 1d und 1 e, wobei in Stufe 1b Natriumethylat anstelle von Natriummethylat verwendet wird. Die Verbindung (If) wird als farbloser Schaum isoliert. α_{D} = +226° (CHCl₃). ¹H-NMR: 8,10 (s, 1H, HC=N), 7,71 (s, 1H, NOH), 7,47 (d, 2H, J = 8,2 , H-3'), 7,19 (d, 2H, J = 8,2 , H-2'), 5,78 (s, 1 H, H-4), 4,38 (d, 1H, J = 7,1, H-11), 3,58 (d, 1H, J = 9,3, CH₂O), 3,55 (m, 2H, CH₂H₅), 3,25 (d, 1H, J = 9,3, CH₂O), 2,17 (s, 1 H, OH), 1,25 (t, 3H, CH₂H₅), 0,52 (s, 3H, H-18).

### Beispiel 5

### 4-[17β-Methoxy-17α-(ethoxymethyl)-3-oxoestra-4,9-dien-11β-yl]benzaldehyd-1E-oxim (Ig)

Die Herstellung der Verbindung (Ig) erfolgt gemäß Beispiel 1, Stufe b, wobei Natriumethylat anstelle von Natriummethylat verwendet wird, und gemäß Beispiel 2, Stufen 2a, 2b, 2c und 2d.
Schmp. 90 bis 95 °C tert.-Butylmethylether). α_{D} = +177° (CHCl₃). ¹H-NMR: 8,10 (s, 1H, HC=N), 7,60 (s, 1 H, NOH), 7,47 (d, 2H, J = 8,1, H-3'), 7,24 (d, 2H, J = 8,1, H-2'), 5,79 (s, 1H, H-4), 4,37 (d, 1H, J = 7,3, H-11), 3,63 (d, 1H, J = 10,8, CH₂O), 3,55 (m, 2H, C₂H₅), 3,44 (d, 1H, J = 10,8 , CH₂O), 3,26, 3,22 (2s, je 3H, OCH₃), 1,27 (t, 3H, C₂H₅), 0,54 (s, 3H, H-18).

### Beispiel 6

### 4-[17β-Hydroxy-17α-(isopropyloxymethyl)-3-oxoestra-4,9-dien-11β-yl]benzaldehyd-1E-oxim (Ih)

Die Herstellung der Verbindung (Ih) erfolgt gemäß Beispiel 1, Stufen 1b, 1 c, 1d und 1 e, wobei in Stufe 1b Natriumisopropylat anstelle von Natriummethylat verwendet wird.
Schmp. 192 bis 196 °C (Zersetzung; Diethylether). α_{D} = +186° (CHCl₃). ¹H-NMR: 8,10 (s, 1 H, HC=N), 8,08 (s, 1 H, NOH), 7,48 (d, 2H, J = 8,1 , H-3'), 7,19 (d, 2H, J = 8,1 , H-2'), 5,79 (s, 1H, H-4), 4,38 (d, 1 H, J = 6,6, H-11), 3,6 (m, CH₂), 3,59 (d, 1 H, J = 9,3, CH₂O), 3,23 (d, 1 H, J = 9,3, CH₂O), 3,02 (s, 1 H, OH), 1,21 (m, 6H, 2xCH₃), 0,52 (s, 3H, H-18).

### Beispiel 7

### 4-[17β-Methoxy-17α-(isopropyloxymethyl)-3-oxoestra-4,9-dien-11β-yl]benzaldehyd-1E-oxim (Ii)

Die Herstellung der Verbindung (Ii) erfolgt gemäß Beispiel 1, Stufe b mit Natriumethylat anstelle von Natriummethylat und gemäß Beispiel 2, Stufen 2a, 2b, 2c und 2d.
Schmp. 143°C (Zersetzung; Aceton/Hexan). α_{D} = +199° (CHCl₃). ¹H-NMR: 8,10 (s, 1 H, HC=N), 8,0 (s, 1 H, NOH), 7,48 (d, 2H, J = 8,4, H-3'), 7,21 (d, 2H, J = 8,1, H-2'), 5,79 (s, 1H, H-4), 4,37 (d, 1 H, J = 6,9, H-11), 3,62 (d, 1 H, J = 10,5, CH₂O), 3,59 (m, 2H, OCH(CH₃)₂), 3,43 (d, 1H, J = 10,2 , CH₂O), 3,26 (s, 3H, OCH₃), 1,22 (t, 3H, C₂H₅), 0,54 (s, 3H, H-18).

### Beispiel 8

### 4-[17β-Hydroxy-17α-(ethylthiomethyl)-3-oxoestra-4,9-dien-11β-yl]benzaldehyd-1E-oxim (Ij)

Die Herstellung der Verbindung (Ij) erfolgt gemäß Beispiel 1, Stufe 1b, 1 c, 1d und 1 e, wobei in Stufe 1b Natriumthioethylat anstelle von Natriummethylat verwendet wird.
Schmp. 132 bis 137°C. α_{D} = +165° (CHCl₃). ¹H-NMR: 8,10 (s, 1H, HC=N), 7,93 (s, 1 H, NOH), 7,49 (d, 2H, J = 8,4 , H-3'), 7,19 (d, 2H, J = 8,4 , H-2'), 5,79 (s, 1 H, H-4), 4,42 (d, 1 H, J = 7,2, H-11), 2,95 (d, 1 H, J = 13,2, CH₂S), 2,90 (s, 3H, OH), 2,71 (d, 1 H, J = 12,9, CH₂S), 2,6 (m, 2H, SCH₂-), 1,29 (t, 3H, SCH₂CH₃),0,56 (s, 3H, H-18).

### Beispiel 9

### 4-{17β-Hydroxy-17α-(1,1,1-trifluorethoxymethyl)-3-oxoestra-4,9-dien-11β-yl]benzaldehyd-1E-oxim (Ik)

Die Herstellung der Verbindung (Ik) erfolgt gemäß Beispiel 1, Stufe 1b, 1c, 1d und 1e, wobei in Stufe 1b 1,1,1-Trifluorethanol und Kalium-tert.-butanolat anstelle von Natriummethylat verwendet wird.
Schmp. 132 bis 136°C (Diethylether). α_{D} = +182° (CHCl₃). ¹H-NMR: 8,10 (s, 1 H, HC=N), 7,60 (s, 1 H, NOH), 7,49 (d, 2H, J = 8,4 , H-3'), 7,20 (d, 2H, J = 8,1 , H-2'), 5,79 (s, 1 H, H-4), 4,41 (d, 1 H, J = 7,2, H-11), 3,95 (m, 2H, OCH₂CF₃ ), 3,92 (d, 1 H, J = 8,7, CH₂O), 3,82 (d, 1 H, J = 9,0, CH₂O), 0,55 (s, 3H, H-18).

## Patentansprüche

1. Verfahren zur Herstellung von 4-(17α-substituierten-3-oxoestra-4,9-dien-11β-yl)benzaldehyd-(1E oder 1Z)-oximen der allgemeinen Formel (I), 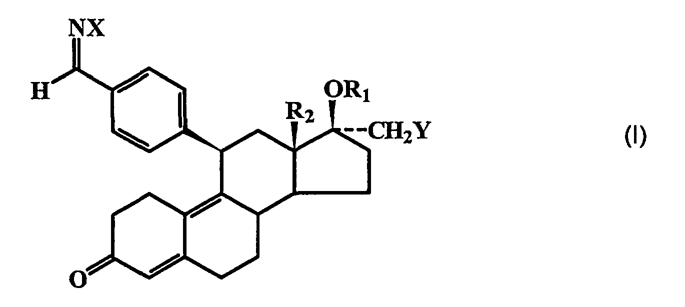 worin R₁ ein Wasserstoffatom, einen C₁₋₆-Alkylrest oder einen CₙF₂ₙ₊₁-Rest bedeutet, R₂ einen C₁₋₄-Alkylrest bedeutet, X eine E- oder Z-ständige OH-Gruppe bedeutet und Y eine OC₁₋₆₋Alkylgruppe, SC₁₋₆-Alkylgruppe oder OCH₂CₙF₂ₙ₊₁-Gruppe bedeutet, wobei n 1, 2 oder 3 ist, **dadurch gekennzeichnet, daß** ein 3,3-Dimethoxy-5α,10α-epoxy-estr-9(11)-en-17-on der Formel (II) 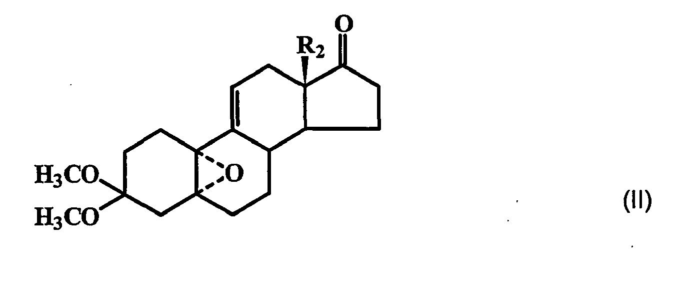 in der R₂ die vorstehend gegebene Bedeutung hat, mit einem aktiven Methylenreagens in einem inerten Lösungsmittel in ein 5α,10α-Epoxy-17(S)-spiroepoxid der Formel (IX) 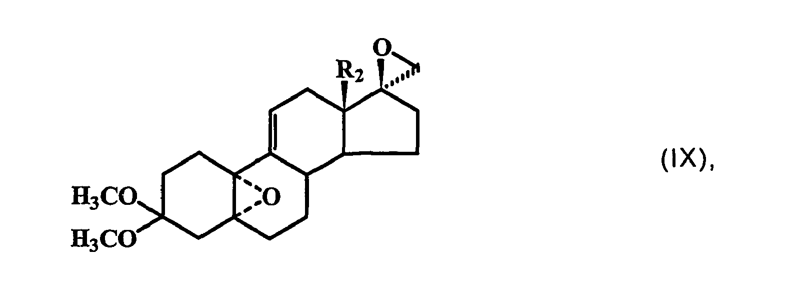 in der R₂ die vorstehend gegebene Bedeutung hat, umgewandelt wird, das nach regio- und stereoselektiver Spaltung der 17-Spiroepoxygruppe durch Alkali- oder Erdalkalialkoholat, durch Alkylmercaptane in Gegenwart von Alkalihydroxiden oder Kaliumtert.-butanolat, durch direkte Spaltung mit Alkalimercaptiden oder mit Perfluoralkylalkoholen in Gegenwart von Alkali in einem inerten Lösungsmittel zu einer 17α-substituierten Verbindung der Formel (X), 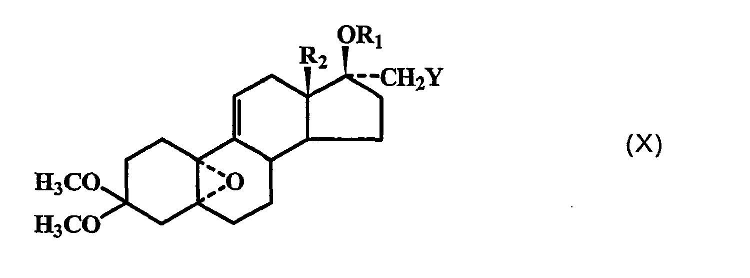 geöffnet wird, in der R₁ ein Wasserstoffatom darstellt, Y eine OC₁₋₆-Alkylgruppe, SC₁₋₆-Alkylgruppe oder OCH₂CₙF₂ₙ₊₁-Gruppe bedeutet, wobei n 1, 2 oder 3 ist und R₂ die vorstehend gegebene Bedeutung hat, die gegebenenfalls durch Umsetzung der 17β-Hydroxylgruppe mit Alkylhalogeniden oder Halogenalkylfluoriden in Gegenwart von starken Basen in einem inerten Lösungsmittel in den 17β-Ether oder 17β-Fluoralkylether der Formel (X) umgewandelt wird, worin R₁ ein C₁₋₆-Alkylrest oder ein CₙF₂ₙ₊₁₋Rest ist, wobei n 1, 2 oder 3 ist, und R₂ und Y die vorstehend gegebene Bedeutung haben, die mit einem 4-Brombenzaldehydketal, Magnesium und Cu(I)Cl bei Temperaturen zwischen -35 °C und Raumtemperatur zu dem entsprechenden 3,3-Dimethoxy-5α-hydroxy-17α-CH₂Y-11β-benzaldehydketal der Formel (XI) 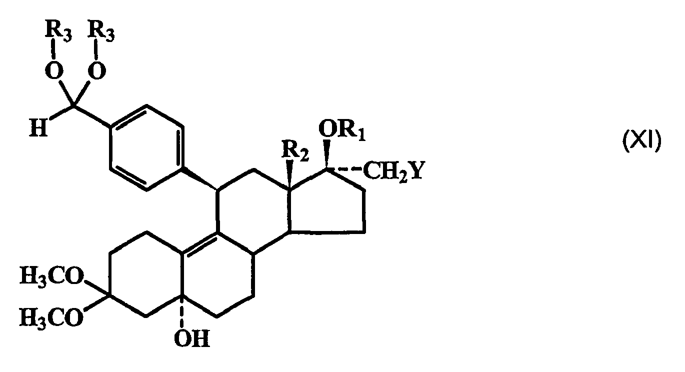 umgesetzt wird, worin R₁ ein Wasserstoffatom, ein C₁₋₆ Alkylrest oder ein CₙF₂ₙ₊₁-Rest ist, wobei n 1, 2 oder 3 ist, R₂ und Y die vorstehend gegebene Bedeutung haben und R₃ für einen Methylrest oder eine Ethylidengruppe steht, die durch saure Hydrolyse der Schutzgruppen in ein 11 β-Benzaldehyd-Derivat der Formel (XII) überführt wird, 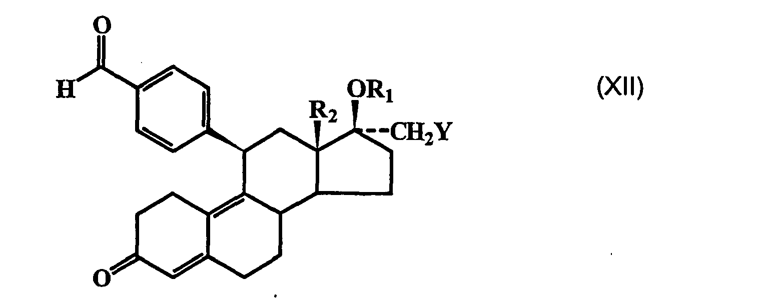 worin R₁, R₂ und Y die vorstehend gegebene Bedeutung haben und die Aldehydfunktion selektiv durch Hydroxylammoniumsalze in ein Gemisch der E/Z-Benzaldoxime der allgemeinen Formel (I), 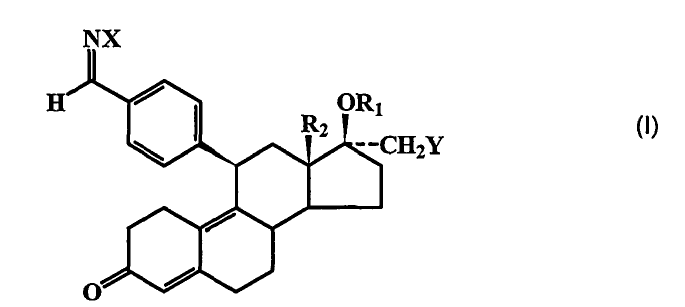 umgewandelt wird, worin X eine E- oder Z-ständige OH-Gruppe bedeutet.

2. Verfahren nach Anspruch 1, **dadurch gekennzeichnet, daß** es den zusätzlichen Verfahrensschritt umfaßt, das Gemisch der E/Z-Benzaldoxime der allgemeinen Formel (I) durch Umkristallisation oder durch Chromatographie in die Einzelkomponenten zu trennen.

3. Verfahren nach Anspruch 1 oder 2, **dadurch gekennzeichnet, daß** R₁ einen C₁₋₃-Alkylrest, vorzugsweise eine Methylgruppe, oder Trifluormethylgruppe bedeutet.

4. Verfahren nach einem der Ansprüche 1 bis 3, **dadurch gekennzeichnet, daß** R₂ einen C₁₋₃-Alkylrest, vorzugsweise eine Methylgruppe, oder Trifluormethylgruppe bedeutet.

5. Verfahren nach einem der Ansprüche 1 bis 4, **dadurch gekennzeichnet, daß** Y einen OC₁₋₃-Alkyl- oder SC₁₋₃-Alkylrest, vorzugsweise eine Methoxy-, Ethoxy-, Isopropyloxy-, Methylthio- oder Ethylthiogruppe, oder Trifluormethoxygruppe bedeutet.

6. Verfahren nach einem der Ansprüche 3 bis 5, **dadurch gekennzeichnet, daß** die Verbindungen
4-[17β-Hydroxy-17α-(methoxymethyl)-3-oxoestra-4,9-dien-11β-yl]benzaldehyd-1 E-oxim,
4-[17β-Hydroxy-17α-(methoxymethyl)-3-oxoestra-4,9-dien-11β-yl]benzaldehyd-1Z-oxim,
4-[17β-Methoxy-17α-(methoxymethyl)-3-oxoestra-4,9-dien-11β-yl]benzaldehyd-1 E-oxim,
4-[17β-Methoxy-17α-(methoxymethyl)-3-oxoestra-4,9-dien-11β-yl]benzaldehyd-1 Z-oxim,
4-[17β-Ethoxy-17α-(methoxymethyl)-3-oxoestra-4,9-dien-11β-yl]benzaldehyd-1 E-oxim,
4-[17β-Hydroxy-17α-(ethoxymethyl)-3-oxoestra-4,9-dien-11β-yl]benzaldehyd-1 E-oxim,
4-[17β-Methoxy-17α-(ethoxymethyl)-3-oxoestra-4,9-dien-11β-yl]benzaldehyd-1 E-oxim,
4-[17β-Hydroxy-17α-(isopropyloxymethyl)-3-oxoestra-4,9-dien-11 β-yl]benzaldehyd-1E-oxim,
4-[17β-Methoxy-17α-(isopropyloxymethyl)-3-oxoestra-4,9-dien-11 β-yl]benzaldehyd-1E-oxim,
4-[17β-Hydroxy-17α-(ethylthiomethyl)-3-oxoestra-4,9-dien-11β-yl]benzaldehyd-1E-oxim und
4-{17β-Hydroxy-17α-(1,1,1-trifluorethoxymethyl)-3-oxoestra-4,9-dien-11β-yl]benzaldehyd-1E-oxim hergestellt werden.

## Claims

1. Process for the production of 4-(17α-substituted-3-oxoestra-4,9-dien-11β-yl)benzaldehyde-(1E or 1Z)-oximes of general formula (I), 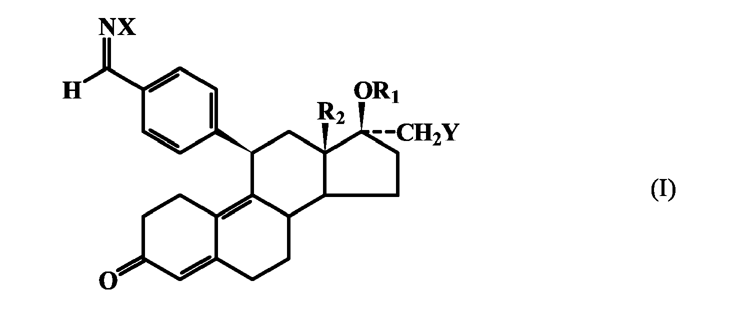 in which R₁ means a hydrogen atom, a C₁₋₆-alkyl radical or a CₙF₂ₙ₊₁ radical, R₂ means a C₁₋₄-alkyl radical, X means an OH group in E- or Z-position, and Y means an OC₁₋₆-alkyl group, an SC₁₋₆-alkyl group or an OCH₂CₙF₂ₙ₊₁ group, whereby n is 1, 2 or 3, **characterized in that** a 3,3-dimethoxy-5α,10α-epoxy-estr-9(11)-en-17-one of formula (II) 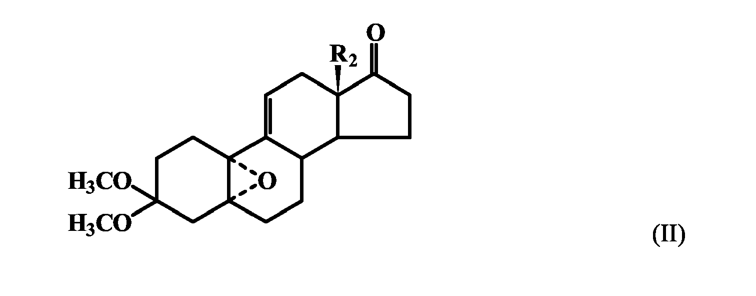 in which R₂ has the above-indicated meaning, is converted with an active methylene reagent in an inert solvent into a 5α,10α-epoxy-17(S)-spiroepoxide of formula (IX) 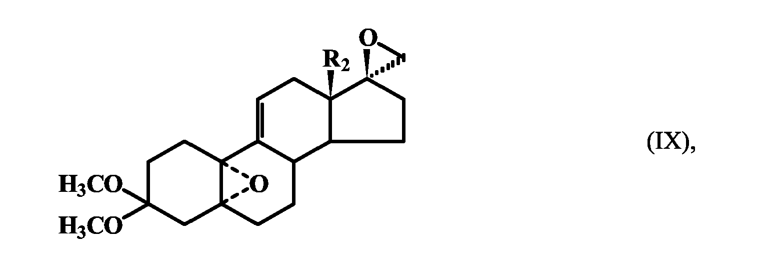 in which R₂ has the above-indicated meaning, which, after regio- and stereoselective cleavage of the 17-spiroepoxy group by alkali or alkaline-earth alcoholate, by alkylmercaptans in the presence of alkali hydroxides or potassium-tert-butanolate, by direct cleavage with alkali mercaptides or with perfluoroalkyl alcohols in the presence of alkali in an inert solvent, is opened to a 17α-substituted compound of formula (X) 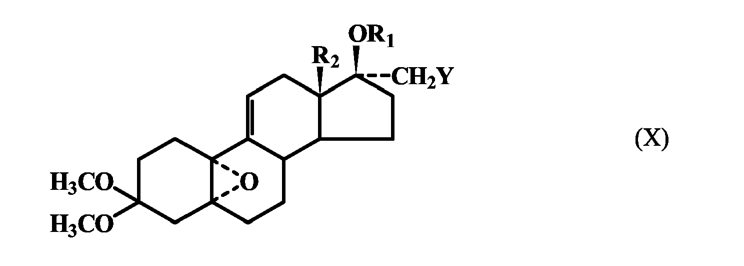 in which R₁ represents a hydrogen atom, Y means an OC₁₋₆-alkyl group, an SC₁₋₆-alkyl group or an OCH₂CₙF₂ₙ₊₁ group, whereby n is 1, 2 or 3, and R₂ has the above-indicated meaning, which optionally is converted by reaction of the 17β-hydroxyl group with alkyl halides or haloalkyl fluorides in the presence of strong bases in an inert solvent into the 17β-ethers or 17β-fluoroalkyl ethers of formula (X), in which R₁ is a C₁₋₆-alkyl radical or a CₙF₂ₙ₊₁ radical, whereby n is 1, 2 or 3, and R₂ and Y have the above-indicated meanings, which is reacted with a 4-bromobenzaldehyde ketal, magnesium and Cu(I)Cl at temperatures of between -35°C and room temperature to form the corresponding 3,3-dimethoxy-5α-hydroxy-17α-CH₂Y-11β-benzaldehyde ketal of formula (XI) 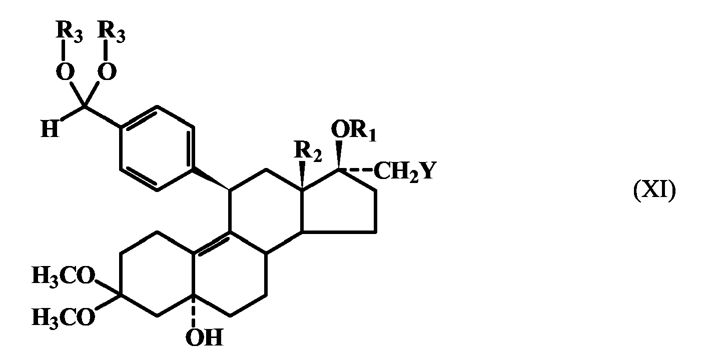 in which R₁ is a hydrogen atom, a C₁₋₆ alkyl radical or a CₙF₂ₙ₊₁ radical, whereby n is 1, 2 or 3, R₂ and Y have the above-indicated meanings, and R₃ stands for a methyl radical or an ethylidene group, which is converted by acid hydrolysis of the protective groups into an 11β-benzaldehyde derivative of formula (XII), 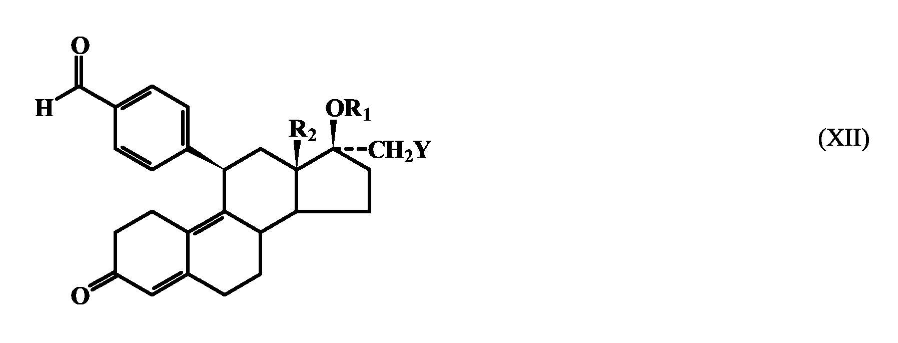 in which R₁, R₂ und Y have the above-indicated meanings, and the aldehyde function is converted selectively by hydroxylammonium salts into a mixture of E/Z-benzaldoximes of general formula (I), 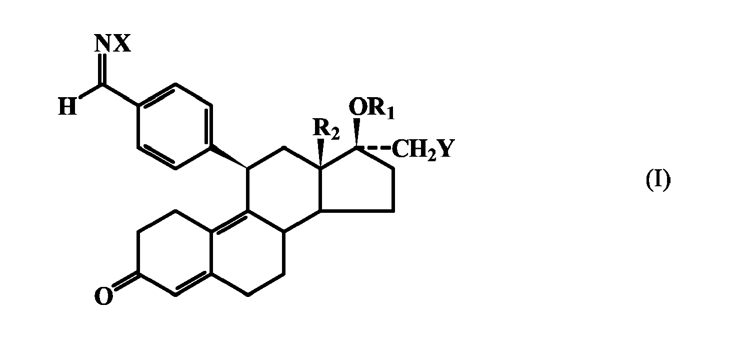 in which X means an OH group in E- or Z-position.

2. Process according to claim 1, **characterized in that** it comprises the additional process steps to separate the mixture of the E/Z-benzaldoximes of general formula (I) by recrystallization or by chromatography into the individual components.

3. Process according to claim 1 or 2, **characterized in that** R₁ means a C₁₋₃-alkyl radical, preferably a methyl group, or a trifluoromethyl group.

4. Process according to one of claims 1 to 3, **characterized in that** R₂ means a C₁₋₃₋alkyl radical, preferably a methyl group, or a trifluoromethyl group.

5. Process according to one of claims 1 to 4, **characterized in that** Y means an OC₁₋₃-alkyl radical or an SC₁₋₃-alkyl radical, preferably a methoxy, ethoxy, isopropyloxy, methylthio or ethylthio group, or a trifluoromethoxy group.

6. Process according to one of claims 3 to 5, **characterized in that** the compounds
4-[17β-Hydroxy-17α-(methoxymethyl)-3-oxoestra-4,9-dien-11β-yl]benzaldehyde-1E-oxime,
4-[17β-Hydroxy-17α-(methoxymethyl)-3-oxoestra-4,9-dien-11β-yl]benzaldehyde-1Z-oxime,
4-[17β-Methoxy-17α-(methoxymethyl)-3-oxoestra-4,9-dien-11β-yl]benzaldehyde-1E-oxime,
4-[17β-Methoxy-17α-(methoxymethyl)-3-oxoestra-4,9-dien-11β-yl]benzaldehyde-1Z-oxime,
4-[17β-Ethoxy-17α-(methoxymethyl)-3-oxoestra-4,9-dien-11β-yl]benzaldehyde-1E-oxime,
4-[17β-Hydroxy-17α-(ethoxymethyl)-3-oxoestra-4,9-dien-11β-yl]benzaldehyde-1E-oxime,
4-[17β-Methoxy-17α-(ethoxymethyl)-3-oxoestra-4,9-dien-11β-yl]benzaldehyde-1E-oxime,
4-[17β-Hydroxy-17α-(isopropyloxymethyl)-3-oxoestra-4,9-dien-11β-yl]benzaldehyde-1E-oxime,
4-[17β-Methoxy-17α-(isopropyloxymethyl)-3-oxoestra-4,9-dien-11β-yl]benzaldehyde-1E-oxime,
4-[17β-Hydroxy-17α-(ethylthiomethyl)-3-oxoestra-4,9-dien-11β-yl]benzaldehyde-1 E-oxime and
4-{17β-Hydroxy-17α-(1,1,1-trifluoroethoxymethyl)-3-oxoestra-4,9-dien-11β-yl]benzaldehyde-1E-oxime
are produced.

## Revendications

1. Procédé de production de 4-(3-oxoestra-4,9-dién-11β-yl)benzaldéhyd(1E ou 1Z)-oximes substitués en 17a de formule générale (I), 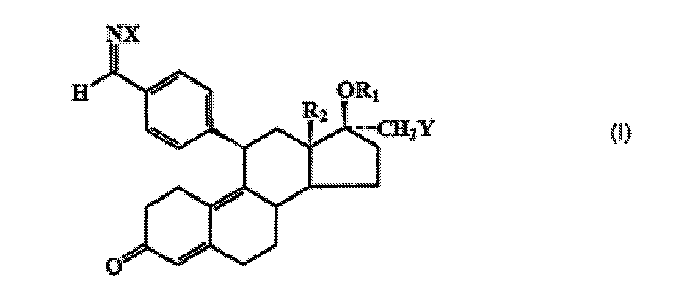 dans laquelle R₁ représente un atome d'hydrogène, un groupement alkyle en C₁₋₆, ou un groupement CₙF₂ₙ₊₁, R₂ représente un groupement alkyle en C₁₋₄, X représente un groupe OH en configuration E ou Z et Y représente un groupement OC₁₋₆-alkyle, un groupement SC₁₋₆-alkyle ou un groupement OCH₂CₙF₂ₙ₊₁, moyennant quoi n est égal à 1, 2 ou 3, **caractérisé en ce qu'on** transforme un 3,3-diméthoxy-5α,10α-époxy-estr-9(11)-én-17-one de formule (II), 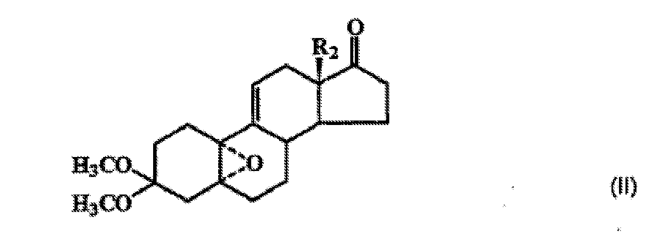 dans laquelle R₂ a la signification mentionnée précédemment, avec un réactif méthylène actif dans un solvant inerte en un 5α,10α-époxy-17(S)-spiroépoxyde de formule (IX) 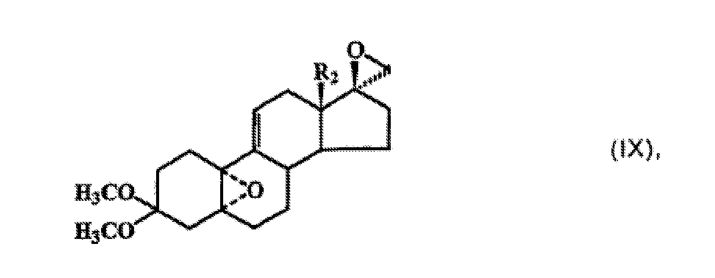 dans laquelle R₂ a la signification mentionnée précédemment, qui après dissociation régio- et stéréosélective du groupe 17-spiroépoxy par de l'alcoolate d'un alcalin ou alcalino-terreux, par de l'alkylmercaptane en présence d'hydroxydes alcalins ou de tert-butanolate de potassium, est ouvert par scission directe avec des thiolates alcalins ou avec des perfluoroalkyl-alcools en présence d'un alcalin dans un solvant inerte, pour former un composé substitué en 17α de formule (X) 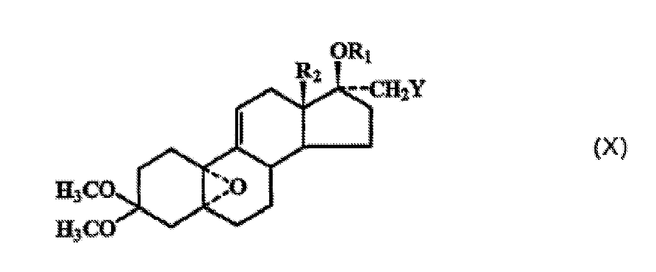 dans laquelle R₁ représente un atome d'hydrogène, Y représente un groupement OC₁₋₆-alkyle, un groupement SC₁₋₆-alkyle ou un groupement OCH₂CₙF₂ₙ₊₁, n étant égal à 1, 2 ou 3, et R₂ a la signification mentionnée précédemment, qu'on transforme éventuellement par réaction du groupe 17β-hydroxyle avec des alkylhalogénures ou des halogène-alkylfluorures en présence de bases fortes dans un solvant inerte en le 17β-éther ou 17β-fluoroalkyléther de la formule (X), dans laquelle R₁ est un groupement C₁₋₆₋alkyle ou un groupement CₙF₂ₙ₊₁, n étant égal à 1, 2 ou 3, et R₂ et Y ont la signification mentionnée précédemment, qu'on fait réagir ensuite avec du 4-bromobenzaldéhyde cétal, du magnésium et du Cu(I)Cl à des températures entre -35°C et la température ambiante, pour former le 3,3-diméthoxy-5α-hydroxy-17α-CH₂Y-11β-benzaldéhyde cétal de formule (XI) 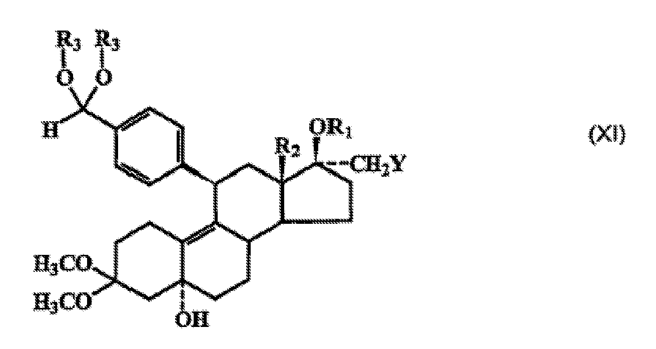 dans laquelle R₁ représente un atome d'hydrogène, un groupement alkyle en C₁₋₆ ou un groupement CₙF₂ₙ₊₁, n étant égal à 1, 2 ou 3, R₂ et Y ont la signification mentionnée précédemment et R₃ représente un groupe méthyle ou un groupe éthylidène, qui est transformé par hydrolyse acide des groupes protecteurs en un dérivé de 11 β-benzaldéhyde de formule (XII), 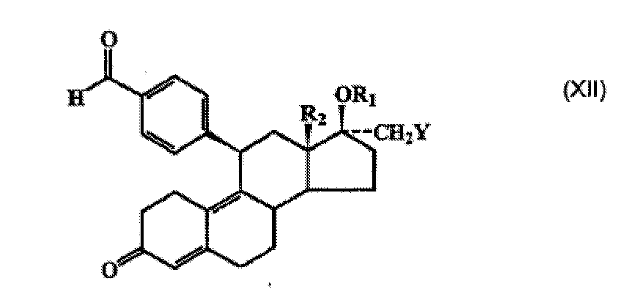 dans laquelle R₁, R₂ et Y ont la signification mentionnée précédemment et on transforme la fonction aldéhyde sélectivement par des sels d'hydroxylammonium en un mélange de benzaldéhydoximes de formule générale (I), 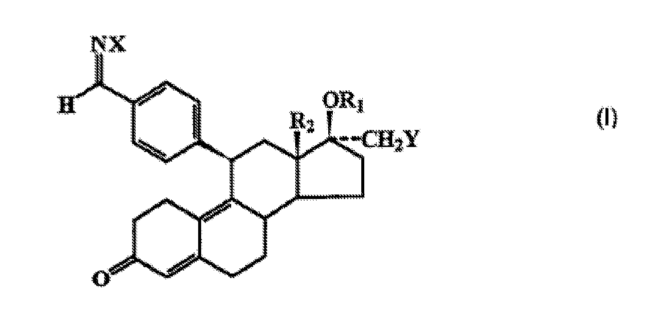 dans laquelle X représente un groupement OH en configuration E ou Z.

2. Procédé selon la revendication 1, **caractérisé en ce qu'**il comporte une étape supplémentaire consistant à séparer le mélange des benzaldéhydoximes E/Z de formule générale (I) en ses différentes composantes par recristallisation ou par chromatographie.

3. Procédé selon la revendication 1 ou 2, **caractérisé en ce que** R₁ représente un groupement alkyle en C₁₋₃, de préférence un groupe méthyle, ou un groupe trifluorométhyle.

4. Procédé selon l'une des revendications 1 à 3, **caractérisé en ce que** R₂ représente un groupement alkyle en C₁₋₃, de préférence un groupe méthyle, ou un groupe trifluorométhyle.

5. Procédé selon l'une des revendications 1 à 4, **caractérisé en ce que** Y représente un groupement OC₁₋₃-alkyle ou un groupement SC₁₋₃-alkyle, de préférence un groupe méthoxy, éthoxy, isopropyloxy, méthylthio ou éthylthio, ou un groupe trifluorométhoxy.

6. Procédé selon l'une des revendications 3 à 5, **caractérisé en ce que** les composés produits sont les suivants :
4-[17β-hydroxy-17α-(méthoxyméthyl)-3-oxoestra-4,9-dién-11β-yl]benzaldéhyd-1 E-oxime,
4-[17β-hydroxy-17α-(méthoxyméthyl)-3-oxoestra-4,9-dién-11β-yl]benzaldéhyd-1 Z-oxime,
4-[17β-méthoxy-17α-(méthoxyméthyl)-3-oxoestra-4,9-dién-11β-yl]benzaldéhyd-1 E-oxime,
4-[17β-méthoxy-17α-(méthoxyméthyl)-3-oxoestra-4,9-dién-11β-yl]benzaldéhyd-1 Z-oxime,
4-[17β-éthoxy-17α-(méthoxyméthyl)-3-oxoestra-4,9-dién-11β-yl]benzaldéhyd-1 E-oxime,
4-[17β-hydroxy-17α-(éthoxyméthyl)-3-oxoestra-4,9-dién-11β-yl]benzaldéhyd-1 E-oxime,
4-[17β-méthoxy-17α-(éthoxyméthyl)-3-oxoestra-4,9-dién-11β-yl]benzaldéhyd-1 E-oxime,
4-[17β-hydroxy-17α-(isopropyloxyméthyl)-3-oxoestra-4,9-dién-11β-yl]benzaldéhyd-1 E-oxime,
4-[17β-méthoxy-17α-(isopropyloxyméthyl)-3-oxoestra-4,9-dién-11β-yl]benzaldéhyd-1 E-oxime,
4-[17β-hydroxy-17α-(éthylthiométhyl)-3-oxoestra-4,9-dién-11β-yl]benzaldéhyd-1 E-oxime et
4-[17β-hydroxy-17α-(1,1,1-trifluoroéthoxyméthyl)-3-oxoestra-4,9-dién-11β-yl]benzaldéhyd-1 E-oxime.
